# EUROPEAN PATENT APPLICATION

(11) **EP 3 173 020 A1**
(43) Date of publication of application: **31.05.2017**
(21) Application number: 16198605.4
(22) Date of filing: 14.11.2016
(51) Int. Cl.: A61B 5/0404, A61B 5/0408, A61B 5/0428

(54) **MOBILE ELECTROCARDIOGRAPH APPARATUS**

(30) Priority: 24.11.2015 JP 2015228944
(71) Applicant: UNION TOOL CO., Tokyo 140-0013 (JP)
(72) Inventor: Shinozaki, Ryo, Tokyo 140-0013 (JP); Ogami, Hisoshi, Tokyo 140-0013 (JP)
(74) Representative: Hoffmann Eitle

(57) **Abstract**

The present invention provides a mobile electrocardiograph (ECG) apparatus in which: there is no need to provide a monitor or other display unit to an ECG unit, allowing the apparatus to be commensurately reduced in size and weight; the cost of operation is reduced through the use of a handheld electrode unit; and a body-surface-adhering electrode can be used in only cases when low-noise tests are required, thus promoting ease of handling. Provided is a mobile ECG apparatus for acquiring an electrocardiographic signal upon electrodes being brought into contact with a body surface, wherein the mobile ECG apparatus is characterized by comprising an ECG unit (1) and an electrode unit that includes a plurality of induction electrodes, the ECG unit (1) being configured so as to acquire an electrocardiographic signal from the body surface potential via the electrode unit, and also being configured so as to be capable of wirelessly transmitting the electrocardiographic signal to an analyzer; the ECG unit (1) and the electrode unit furthermore being provided so as to be capable of being attached to and detached from each other by a plurality of connection parts; and the plurality of connection parts being provided with induction-electrode contact points for respectively connecting to the plurality of induction electrodes.

## Description

### TECHNICAL FIELD

The present invention relates to a mobile electrocardiograph (ECG) apparatus.

### BACKGROUND ART

Auscultation, medical interviews, resting electrocardiographic tests, and the like are carried out in health examinations in order to detect cardiovascular disease; however, it is difficult to detect all cardiovascular disease from these alone. For example, there are cases in which paroxysmal arrhythmia or the like is not manifested in auscultation or electrocardiographic tests that are performed in a short time period. In order to ascertain such symptoms that present suddenly, there are mobile ECG apparatuses, such as that disclosed in Patent Document 1, with which it is possible to perform electrocardiographic tests immediately when a subject experiences symptoms.

The mobile ECG apparatus disclosed in Patent Document 1 is configured so as to acquire an electrocardiographic signal when a grip portion including a negative electrode and a contact portion including a positive electrode are brought into contact with the palms of the two hands of a subject, the grip portion being designed so as to be held in the hand, or when a grip portion including a negative electrode is gripped in the right hand of the subject and a contact portion including a positive electrode is brought into contact with the lower-left quadrant of the abdomen.

A mobile ECG of such description presents advantages in terms of obviating disposable electrodes, and in terms of the very low cost of operation; however, this configuration necessitates a display unit for displaying the measurement results, a memory, and a large battery for driving these devices. Therefore, a mobile ECG having the above configuration presents a drawback in being heavy, in that the muscles of the two hands or of the right hand supporting the ECG body of the apparatus are actuated, and in that contamination by myoelectric noise readily occurs.

A configuration may be adopted in which a Holter ECG is used (to avoid having to hold the apparatus in the hands) together with disposable electrodes to reduce the myoelectric noise; however, in such a case, a drawback is presented in terms of the increased cost of operation.

### [Prior-art Documents]

[Patent Document 1] JP-A 2005-468

### DISCLOSURE OF THE INVENTION

### PROBLEMS TO BE SOLVED BY THE INVENTION

In view of the above, the present invention was contrived in order to provide a mobile electrocardiograph (ECG) apparatus in which: there is no need to provide a monitor or other display unit to an ECG unit, allowing the apparatus to be commensurately reduced in size and weight; the cost of operation is reduced through the use of a handheld electrode unit; and a body-surface-adhering electrode can be used only in cases when low-noise tests are required, thus promoting ease of handling.

### MEANS FOR SOLVING THE ABOVEMENTIONED PROBLEMS

The main points of the present invention are described below with reference to the attached drawings.

The present invention relates to a mobile electrocardiograph (ECG) apparatus for acquiring an electrocardiographic signal upon electrodes being brought into contact with a body surface, wherein the mobile ECG apparatus is characterized by comprising an ECG unit 1 and an electrode unit that includes a plurality of induction electrodes, the ECG unit 1 being configured so as to acquire an electrocardiographic signal from the body surface potential via the electrode unit, and also being configured so as to be capable of wirelessly transmitting the electrocardiographic signal to an analyzer; the ECG unit 1 and the electrode unit furthermore being provided so as to be capable of being attached to and detached from each other by a plurality of connection parts; and the plurality of connection parts being provided with induction-electrode contact points for respectively connecting to the plurality of induction electrodes.

The present invention also relates to a mobile ECG apparatus characterized in that: the connection parts are configured from connecting members 2 provided to the ECG unit 1, and to-be-connected members 4 provided to the electrode unit; and the induction-electrode contact points 2' are provided to at least the connecting members 2.

The present invention also relates to a mobile ECG apparatus characterized in that the ECG unit 1 is provided with signal processing means for converting the body surface potential to an electrocardiographic signal, sampling means for sampling the electrocardiographic signal obtained by the signal processing means, and transmission means for wirelessly transmitting the sampled electrocardiographic signal to an analyzer.

The present invention also relates to a mobile ECG apparatus characterized in that the electrode unit is a handheld electrode unit 3 that can be held in the hands.

The present invention also relates to a mobile ECG apparatus characterized in that the handheld electrode unit 3 is provided in a state such that at least two induction electrodes A, B are set apart from each other.

The present invention also relates to a mobile ECG apparatus characterized in that the at least two induction electrodes A, B are separated by a distance of 100 mm or more.

The present invention also relates to a mobile ECG apparatus characterized in that: the handheld electrode unit 3 is a substrate that can be held in the hands; and the at least two induction electrodes A, B are provided on the two side-end surfaces of the substrate so as to sandwich the connection parts.

The present invention also relates to a mobile ECG apparatus characterized in that: the substrate upper surface, which is substantially perpendicular to the right-side end surface on which is provided one induction electrode A from among the induction electrodes A, B, is provided with a cavity 7 for induction at the thumb at a position where contact is made by the thumb of the right hand; and the substrate lower surface, which is on the opposite side from the substrate upper surface, is provided with a cavity 8 for induction at the middle finger, the cavity 8 contacting the middle finger of the right hand.

The present invention also relates to a mobile ECG apparatus characterized in that the right-side end surface and the left-side end surface are both set in a shape so as to bend convexly outward.

The present invention also relates to a mobile ECG apparatus characterized in that the electrode unit is a body-surface-adhering electrode 11 that can adhere to the body surface.

### EFFECT OF THE INVENTION

Because the present invention is configured as described above, it is possible to provide a mobile ECG apparatus in which: there is no need to provide a monitor or other display unit to an ECG unit, allowing the apparatus to be commensurately reduced in size and weight; the cost of operation is reduced through the use of a handheld electrode unit; and a body-surface-adhering electrode can be used only in cases when low-noise tests are required, thus promoting ease of handling.

### BRIEF DESCRIPTION OF THE DRAWINGS

FIG. 1 is a configurational schematic diagram of the present example;
FIG. 2 is a schematic perspective view of the present example;
FIG. 3 is a configurational schematic diagram of other example 1;
FIG. 4 is a schematic perspective view of other example 1;
FIG. 5 is a schematic front view of other example 1;
FIG. 6 is a schematic side view of other example 1;
FIG. 7 is a schematic diagram showing other example 1 in a state of use;
FIG. 8 is a schematic front view showing other example 1 in a state of use;
FIG. 9 is a schematic perspective view of other example 2;
FIG. 10 is a schematic view showing other example 2 in a state of use;
FIG. 11 is a schematic perspective view of other example 3; and
FIG. 12 is a schematic view showing other example 3 in a state of use.

### BEST MODE FOR CARRYING OUT THE INVENTION

Preferred examples of the present invention are concisely described below with reference to the drawings by indicating the effects of the present invention.

In cases when a handheld electrode unit 3 is used, an ECG unit 1 acquires an electrocardiographic signal in a state in which induction electrodes A, B of a handheld electrode unit 3 on which the ECG unit 1 is mounted are brought into contact with the palms of the two hands or the like of a subject such that the handheld electrode unit 3 is being gripped, the acquired electrocardiographic signal is transmitted to a personal computer (referred to below as a "PC") or other analyzer, and the electrocardiographic signal is then analyzed.

Specifically, it is possible to transmit the electrocardiographic signal to a separate analyzer for analysis and display. This obviates the need to provide an electrocardiographic analysis mechanism, or a monitor or other display unit, to the apparatus itself, thus making it possible to correspondingly reduce the size and weight of the apparatus and battery. Accordingly, when an electrocardiographic signal is acquired using a handheld electrode unit 3, myoelectric noise is not readily mixed with the electrocardiographic signal.

Therefore, adopting a configuration in which myoelectric noise is not readily mixed with the electrocardiographic signal while the handheld electrode unit 3 is used makes it possible to easily perform highly precise tests, obviates the need to continuously use a body-surface-adhering electrode (disposable electrode), and correspondingly reduces the cost of operation.

In cases such as when noise is to be reduced as much as possible, it is possible to: attach a body-surface-adhering electrode 11, as the electrode unit, to an induction-electrode contact point on the ECG unit 1 (i.e., to connect a connecting member 2 of the ECG unit 1 and a to-be-connected member 4 of the body-surface-adhering electrode 11) without connecting the ECG unit 1 to the handheld unit 3; and bond the body-surface-adhering electrode 11 to the chest of the subject, whereby an electrocardiographic signal is acquired from the body surface potential obtained via the body-surface-adhering electrode 11.

Specifically, the present invention is configured so as to be capable of connecting a detachable electrode (or a contact part of such an electrode) to an electrode contact point on the ECG unit, thereby making it possible for an operator to properly use the apparatus in accordance with symptoms and/or conditions of a body.

Additionally, although only one type of electrocardiographic signal can be obtained in cases when the electrocardiographic signals are acquired using a handheld electrode unit 3 comprising two induction electrodes, adding an additional induction electrode to the ECG unit 1 makes it possible, e.g., to acquire electrocardiographic signals that cannot be obtained from only the two hands and reduce electrocardiographic noise. Therefore, it is possible to perform more highly precise tests.

### [Examples]

Specific examples of the present invention are described below with reference to the diagrams.

The present example relates to a mobile ECG apparatus for acquiring an electrocardiographic signal upon electrodes being brought into contact with a body surface, wherein: the mobile ECG apparatus comprises an ECG unit 1 and an electrode unit that includes a plurality of induction electrodes; the ECG unit 1 is configured so as to acquire an electrocardiographic signal from the body surface potential via the electrode unit, and is also configured so as to be capable of wirelessly transmitting the electrocardiographic signal to an analyzer; the ECG unit 1 and the electrode unit are furthermore provided so as to be capable of being attached to and detached from each other by a plurality of connection parts; and the plurality of connection parts are provided with induction-electrode contact points for respectively connecting to the plurality of induction electrodes.

Specifically, the connection parts in the present example are configured from connecting members 2 provided to the ECG unit 1 and to-be-connected members 4 provided to the electrode unit, the connecting members 2 additionally functioning as a plurality of induction-electrode contact points 2' for connecting to the plurality of induction electrodes. In the present example, a configuration in which a handheld electrode unit 3 comprising a substrate that can be held in the hands is used, and in which to-be-connected members 4 of the handheld electrode unit 3 and connecting members 2 (induction-electrode contact points 2') of the ECG unit 1 are connected, is described as pertains to a method for acquiring the electrocardiographic signal.

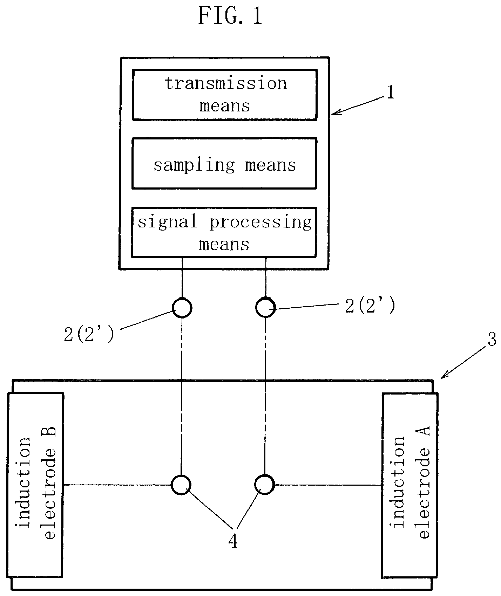

As shown in FIG. 1, the ECG unit 1 is provided with: signal processing means for converting the body surface potential obtained from the electrode unit (handheld electrode unit 3) through the connecting members 2, which are provided with the induction-electrode contact points 2', to an electrocardiographic signal; sampling means for sampling the electrocardiographic signal obtained by the signal processing means; and transmission means for wirelessly transmitting the sampled electrocardiographic signal to an analyzer. The electrocardiographic signal can be analyzed and displayed using a PC or other analyzer once the electrocardiographic signal is transmitted to the analyzer by the transmission means. This obviates the need to provide an electrocardiographic analysis mechanism, or a monitor or other display unit, to the apparatus itself, so that the apparatus and battery can be commensurately reduced in size and weight. Specifically, in the present example, an analog filter is used as the signal processing means and radio waves are used as the transmission means; however, a configuration may be adopted in which a digital filter is used as the signal processing means and infrared communication or the like is used as the transmission means.

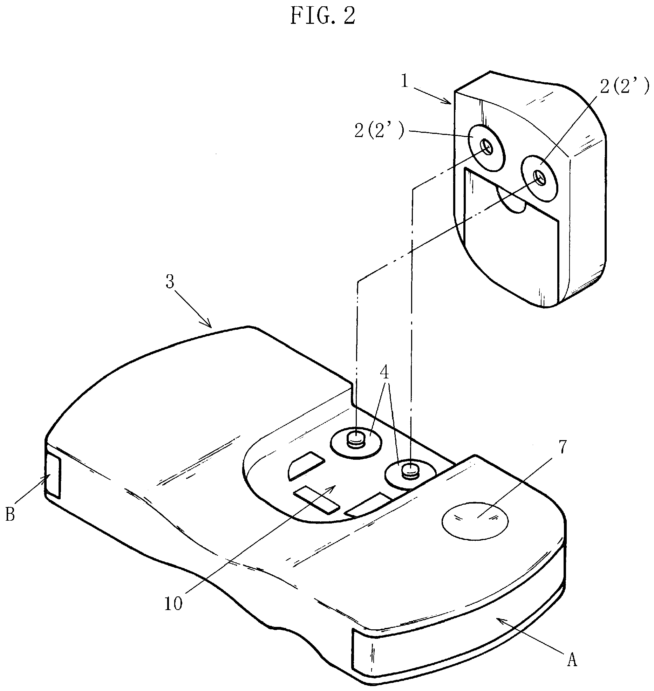

As shown in FIG. 2, a configuration is adopted in which the handheld electrode unit 3 is provided with a concave mating part 10 into which the ECG unit 1 is fitted, and the connecting members 2 (additionally functioning as the induction-electrode contact points 2') provided on the lower surface of the ECG unit 1 and the to-be-connected members 4 provided on the upper surface (inside the concave mating part 10) of the handheld electrode unit 3 are attachably/detachably connected by fitting the ECG unit 1 into the concave mating part 10 to mount the ECG unit 1 on the handheld electrode unit 3. Additionally, a configuration is adopted in which induction-electrode contact points are provided to the to-be-connected members 4 so that the to-be-connected members 4 additionally function as induction-electrode contact points, similarly to the connecting members 2, therefore making it possible to obtain the body surface potential and acquire the electrocardiographic signal by connecting the induction-electrode connection points.

In the present example, a "snap-button-type" mechanism, in which the connecting members 2 of the ECG unit 1 have barrel-shaped concave parts in the central portions thereof and the to-be-connected members 4 of the handheld electrode unit 3 have barrel-shaped convex parts for mating with the barrel-shaped concave parts, is employed as the attachment/detachment mechanism. Causing these barrel-shaped parts to matingly connect makes it possible to acquire the electrocardiographic signal from the body surface potential obtained from induction electrodes A, B. The present invention is not limited to this snap-button-type mechanism; other mechanisms, such as an electroconductive surface fastener or an electroconductive tape, may be employed as long as it is possible to ensure electroconductivity and to easily perform the attachment/detachment operation.

As shown in FIGS. 1 and 2, in the present example, two connecting members 2 (induction-electrode contact points 2') are provided to the ECG unit 1, the connecting members 2 respectively corresponding to two electrodes A, B of the handheld electrode unit 3. The two electrodes A, B of the handheld electrode unit 3 are induction electrodes provided so as to be set apart from each other. In the present example, the induction electrodes A, B are provided on the two side-end surfaces of the substrate so as to sandwich the to-be-connected members 4 (concave mating parts 10). The pair of induction electrodes A, B provided on the side-end surfaces are provided so as to be separated by a distance of 100 mm or more; this separation by a distance of 100 mm or more makes it possible to avoid contact between the two hands, and to precisely measure the difference in body surface potential between the two hands.

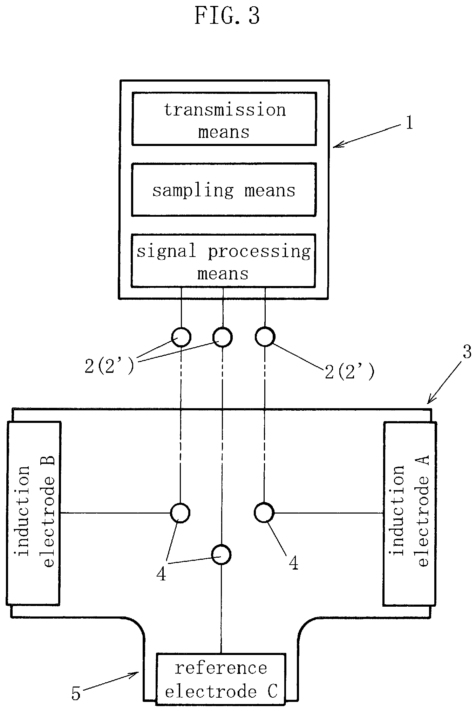

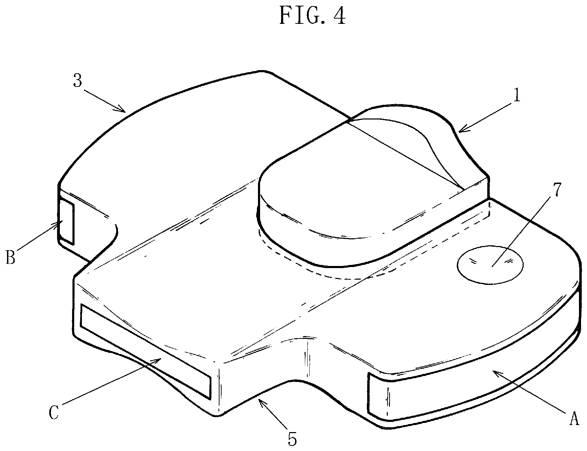

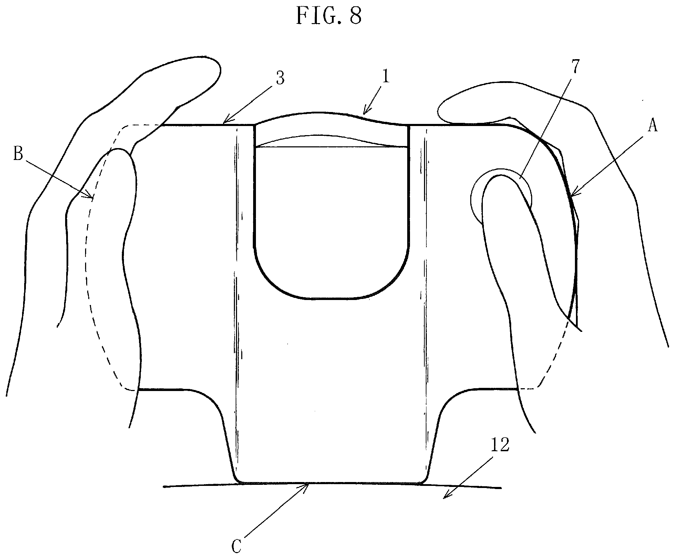

In addition to the pair of induction electrodes A, B provided to the side-end surfaces of the substrate, it is also possible to provide a reference electrode C to a protruding part 5 that protrudes forward from the handheld electrode unit 3, as in other example 1 shown in FIGS. 3 and 4. In this case, a test is performed in a state in which the handheld electrode unit 3 is gripped while the two hands are respectively in contact with the pair of induction electrodes A, B, and in which the reference electrode C is pressed against the chest (indicated by reference symbol 12 in FIG. 8) or the like, as shown in, e.g., FIG. 8; this allows the reference electrode C, which is a single induction electrode, to serve as a reference potential when the difference in potential between the induction electrode A and the induction electrode B is to be obtained. Therefore, in this case, baseline fluctuation of the ECG and other such noise is prevented from contaminating the electrocardiographic signal, and it is possible to more accurately obtain the electrocardiographic signal.

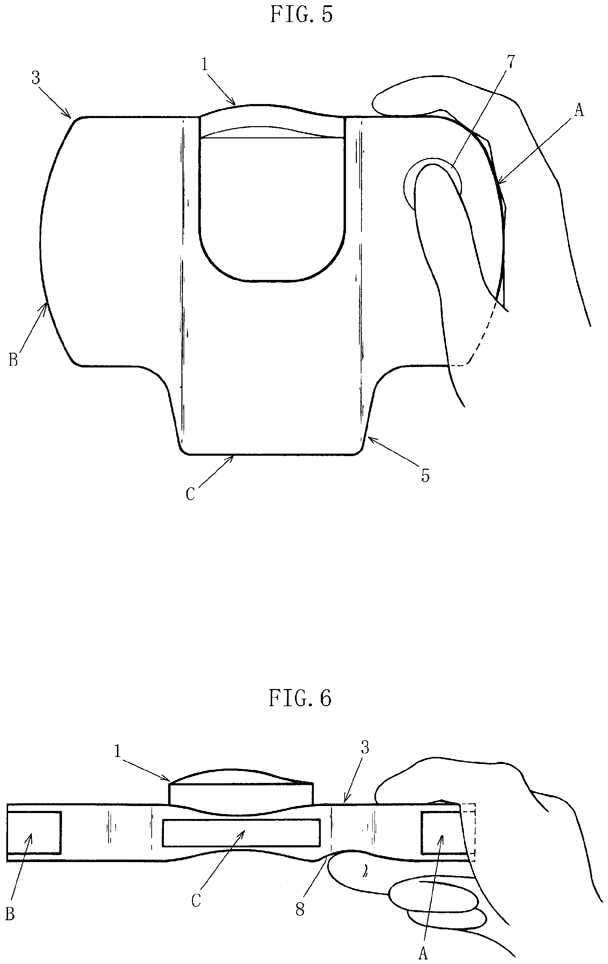

As shown in FIGS. 5 and 6, the substrate upper surface, which is substantially perpendicular to the right-side end surface on which is provided one induction electrode A from among the pair of induction electrodes A, B provided to the side-end surfaces of the handheld electrode unit 3, is provided with a semispherical cavity 7 for induction from the thumb (see FIGS. 4 and 5) at a position contacted by the thumb of the right hand when the handheld electrode unit 3 is gripped in a manner such that the one induction electrode A provided to the right-side end surface comes into contact with the right hand in a range from the index finger to the ball of the thumb. The cavity 7 for induction from the thumb may be ellipsoidal or otherwise non-semispherical in shape as long as induction from the thumb is possible.

Additionally, the substrate lower surface, which is on the opposite side from the substrate upper surface of the handheld electrode unit 3, is provided with an arcuate-groove-shaped cavity 8 for induction from the middle finger at a position so as to contact the middle finger of the right hand when the handheld electrode unit 3 is gripped in a manner such that the one induction electrode A provided to the right-side end surface is in contact with the right hand in a range from the index finger to the ball of the thumb. The cavity 8 for induction from the middle finger is provided along a direction orthogonal to the direction in which the pair of induction electrodes A, B face. The cavity 8 for induction from the middle finger may be configured such that induction is carried out from the ring finger as well as from the middle finger.

Therefore, because the cavity 7 for induction from the thumb and the cavity 8 for induction from the middle finger are provided, it is possible to more reliably bring the right hand into contact with the one induction electrode A in a range from the index finger to the ball of the thumb when the handheld electrode unit 3 is gripped in the right hand, and to grip the handheld electrode unit 3 in a stable state. If the handheld electrode unit 3 can be stably gripped in this manner, it is possible to reduce contamination of the ECG by myoelectricity, and to obtain electrocardiographic measurements having less noise.

The right-side end surface provided with the one induction electrode A with which the right hand comes into contact and the left-side end surface provided with the other induction electrode B facing the opposite direction are both set in a shape so as to bend convexly outward. In the present example and in other example 1, the right-side end surface is similarly set in a shape so as to bend convexly outward.

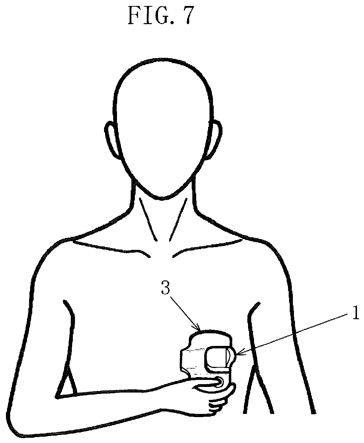

Therefore, as shown in FIG. 7, when the other induction electrode B is pressed on a region between the lower-left side of the chest and the left side of the abdomen while the right hand is held so as to contact the one induction electrode A, it is possible to contact the body surface (skin) without adding more force, and to perform tests correspondingly more easily. Additionally, because it is not necessary to add more force, it is possible to reduce contamination of the ECG by myoelectricity, and to obtain electrocardiographic measurements having even less noise.

Accordingly, in the present example and in other example 1, it is easy to perform tests in an orientation in which the handheld electrode unit 3 is gripped in the right hand such that the palm of the right hand is in contact with the one induction electrode A, and in which the other induction electrode B is pressed against the chest or another location other than the left hand (e.g., the left leg). At such time, a circuit is completed from the location on the body of the subject with which the other induction electrode B is in contact through the heart to the right hand with which the one induction electrode A is in contact, and it is possible to obtain, from the difference in potential between the one induction electrode A and the other induction electrode B, the action potential that accompanies electrical excitation produced during activity of the heart muscle of the subject; this makes it possible to measure the electrocardiographic signal.

In other example 1, the electrode C is described as a reference electrode C; however, a configuration may be adopted in which the electrode C is configured as an induction electrode that is not a reference electrode. There is a possibility that there will be more noise in this case than in a case in which a reference electrode is used, but obtaining the difference in potential between the induction electrode A and the induction electrode C, and the difference in potential between the induction electrode B and the induction electrode C, makes it possible to obtain information about two types of electrocardiographic signals. In this case, increasing the amount of information obtained makes it possible to diagnose a disease from different standpoints.

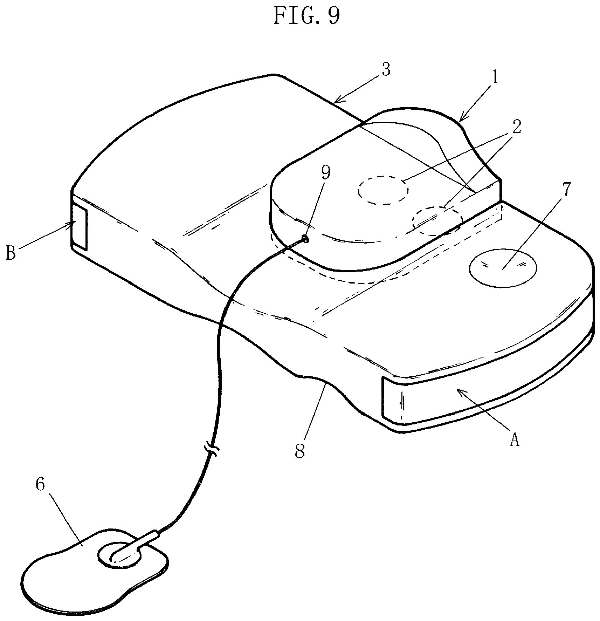

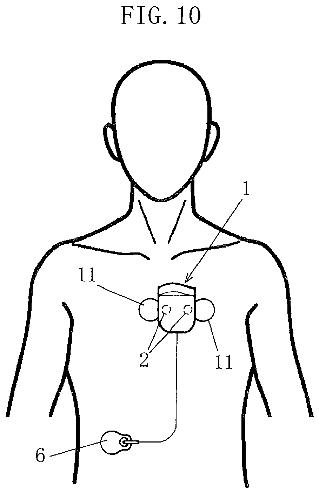

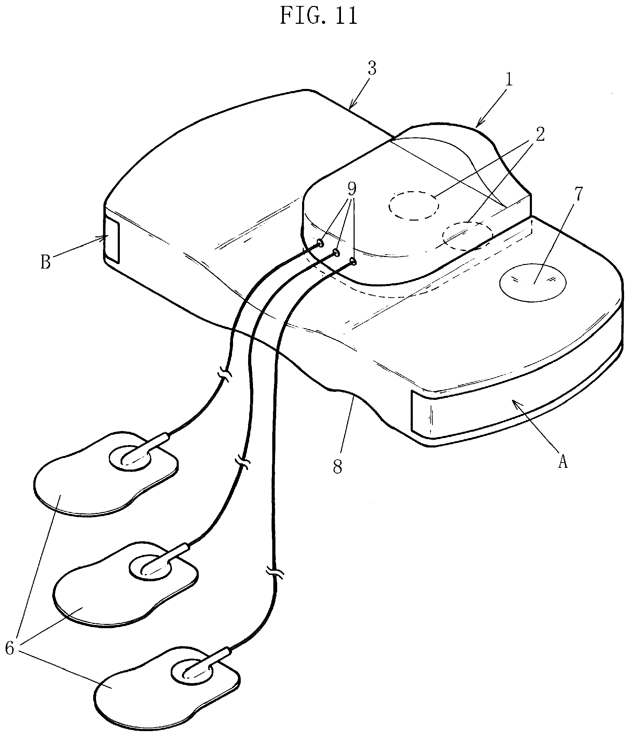

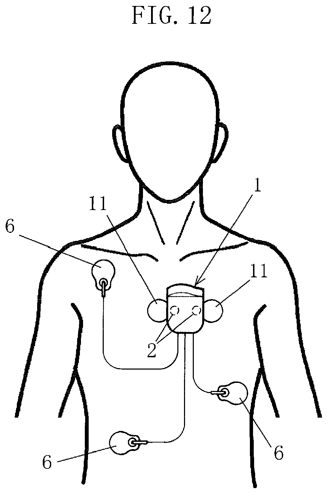

FIGS. 9 and 10 show the configuration of other example 2, and FIGS. 11 and 12 show the configuration of other example 3.

In other example 2 shown in FIG. 9, a configuration is adopted in which: an ECG unit 1 having two connecting members 2 (induction-electrode contact points 2') is fitted into a concave mating part 10 in a handheld electrode unit 3 having two induction electrodes A, B and two to-be-connected members 4 (see FIG. 2); and one sub-electrode 6, which is an induction electrode, is furthermore attached to the ECG unit 1. The sub-electrode 6 is connected to a sub-electrode contact point 9 by inserting a conductive line into the side surface of the ECG unit 1. Specifically, when the ECG unit 1 and the handheld electrode unit 3 are fitted together and gripped in the two hands, the sub-electrode 6 is connected to the sub-electrode contact point 9 by inserting a conductive line into the front side of a side surface of the ECG unit 1. In other example 2, a body-surface-adhering electrode; i.e., a disposable electrode, is employed as the sub-electrode 6. In other example 2, the connected sub-electrode 6 can be used as a reference electrode or the like; therefore, although FIG. 9 shows a handheld electrode unit 3 (corresponding to the handheld electrode unit 3 of the present example) that does not have a reference electrode C, a configuration may be adopted in which a handheld electrode unit 3 is used that does have a reference electrode unit 3 as shown in FIGS. 3-8. In this case, a configuration may be adopted in which the reference electrode C provided to the handheld electrode unit 3 is not used. When the sub-electrode 6 is used, it is possible to prevent baseline fluctuation and other noise from contaminating the electrocardiographic signal to a greater extent than when the reference electrode C provided to the handheld electrode unit 3 is used, and the electrocardiographic signal can be more accurately acquired. Because myoelectricity and other noise is further prevented from contaminating the electrocardiographic signal, as shown in FIG. 10, it is possible to attach two body-surface-adhering electrodes 11 to the connecting members 2 (induction-electrode contact points 2') of the ECG unit 1 in other example 2, instead of to the handheld electrode unit 3, and to then bond the body-surface-adhering electrodes 11 to the left side of the chest, thereby acquiring a body-surface electrocardiographic signal. The one remaining sub-electrode 6 is bonded at, e.g., the lower-right quadrant of the abdomen. In this case, the cost of operation increases due to use of the body-surface-adhering electrodes 11, but the effect of noise caused by myoelectricity from the arms can be reduced; furthermore, when the sub-electrode 6 is used as a reference potential (when the difference in potential between the induction electrodes A, B is measured with this potential as a reference), it is possible to prevent baseline fluctuation and other noise from contaminating the electrocardiographic signal, and to more accurately acquire the electrocardiographic signal. Similarly to the handheld electrode unit 3, the body-surface-adhering electrodes 11 of other example 2 have members to be connected, and a mechanism is employed therein such that the central portions thereof have barrel-shaped convex parts for mating with the barrel-shaped concave parts of the ECG unit 1; therefore, the same ECG unit 1 having the barrel-shaped concave parts in the central portion of the connecting members 2 can easily be attached to and detached from the body-surface-adhering electrodes 11. Although the sub-electrode 6 is bonded at the lower-right quadrant of the abdomen, other locations, such as a point on the torso that is positioned over a bone where there is little muscle, may be used.

A configuration may be adopted such that three or more connecting members 2 (induction-electrode contact points 2') are provided to the lower surface of the ECG unit 1, as shown in FIG. 3; however, in cases when two body-surface-adhering electrodes 11 (disposable electrodes) are connected to such an ECG unit 1, the one connecting member 2 that cannot be connected to the induction electrode or to the reference electrode (sub-electrode 6) goes unused (becomes redundant).

In other example 3 shown in FIG. 11, a configuration is adopted in which an ECG unit 1 having two connecting members 2 (induction-electrode contact points 2') is fitted into the concave mating part 10 of a handheld electrode unit 3 that has two induction electrodes A, B and two to-be-connected members 4, and three sub-electrodes 6 that are induction electrodes are furthermore attached to the ECG unit 1 (i.e., there are a total of five induction electrodes). The sub-electrodes 6 are body-surface-adhering electrodes (disposable electrodes) of the same type as in other example 2. The three sub-electrodes 6 are respectively connected to three sub-electrode contact points 9 by inserting a conductive line into the front side of a side surface of the ECG unit 1. In this case, two of the three sub-electrodes 6 can be used as induction electrodes in a group separate from the induction electrodes A, B of the handheld electrode unit 3, and the remaining sub-electrode 6 can be used as a reference electrode (i.e., a configuration can be adopted in which there are two groups of induction electrodes and one reference electrode), making it possible to acquire the electrocardiographic signal. Thus, increasing the number of sub-electrodes 6 and sub-electrode contact points 9 of the ECG unit 1 and increasing the number of groups of induction electrodes in this manner correspondingly increases the number of types of electrocardiographs that can be obtained from the body surface potential but that cannot be induced using only the handheld electrode unit 3, and makes it possible to perform more precise tests.

As shown in FIG. 12, it is also possible to attach two body-surface-adhering electrodes 11 (disposable electrodes) to the connecting members 2 (induction-electrode contact points 2') of the ECG unit 1 in other example 3, instead of to the handheld electrode unit 3, and then bond the body-surface-adhering electrodes 11 to the left side of the chest, and furthermore to respectively attach the sub-electrodes 6 to the three sub-electrode contact points 9 and then bond the sub-electrodes 6 to sites other than the left side of the chest. In other example 3, a configuration is adopted in which the three sub-electrodes 6 are respectively bonded at positions on the upper-right side of the chest, the left side of the abdomen, and the lower-right quadrant of the abdomen, allowing the body surface potential to be induced from a total of five locations. If the body surface potential from one of the five locations is used as a reference potential, it is possible to acquire two types of electrocardiographic signals from the other four locations while preventing baseline fluctuation as well as contamination of the electrocardiographic signals by noise caused by myoelectricity from the arms. Specifically, using four (two groups) of the five disposable electrodes as induction electrodes and using the remaining one of the five disposable electrodes as a reference electrode makes it possible to acquire more highly precise measurement results.

In the case of other example 3 as well, it is possible to acquire electrocardiographic signals without using a reference electrode. There may be more noise in this case than in a case in which a reference electrode is used, but because it is possible to obtain information about a greater number of types of electrocardiographic signals, diseases can be diagnosed from different standpoints.

Because the present example is configured as described above, the electrocardiographic signal can be analyzed and displayed using a separate PC or other analyzer once the electrocardiographic signal is transmitted to the analyzer. This obviates the need to provide an electrocardiographic analysis mechanism, or a monitor or other display unit, to the apparatus itself, thus making it possible to correspondingly reduce the size and weight of the apparatus and battery. When the electrocardiographic signal is acquired using a handheld electrode unit 3, myoelectric noise is not readily mixed with the electrocardiographic signal.

Therefore, achieving a configuration in which the electrocardiographic signal is not readily contaminated by myoelectric noise while using a handheld electrode unit 3 makes it possible to easily perform highly precise tests, and correspondingly reduces the cost of operation.

Additionally, in cases when noise is to be reduced as much as possible, or in other such situations, it is possible to attach a body-surface-adhering electrode 11 to the connecting members 2 (induction-electrode contact points 2') of the ECG unit 1 without fitting the handheld electrode unit 3 into the ECG unit 1, and bond the body-surface-adhering electrode 11 to the left side of the chest of the subject, thereby acquiring an electrocardiographic signal.

Additionally, providing sub-electrode contact points 9 to the ECG unit 1 and achieving a configuration such that sub-electrodes 6 can be connected makes it possible to acquire electrocardiographic signals that cannot be obtained from only the two hands or a reference potential; therefore, it is possible to perform more highly precise tests.

Accordingly, the present example provides a highly utilitarian mobile ECG apparatus with which it is possible for an operator to properly use the apparatus in accordance with symptoms and/or conditions of a body.

## Claims

1. A mobile electrocardiograph (ECG) apparatus for acquiring an electrocardiographic signal upon electrodes being brought into contact with a body surface, wherein the mobile ECG apparatus is **characterized by** comprising an ECG unit and an electrode unit that includes a plurality of induction electrodes, the ECG unit being configured so as to acquire an electrocardiographic signal from the body surface potential via the electrode unit, and also being configured so as to be capable of wirelessly transmitting the electrocardiographic signal to an analyzer; the ECG unit and the electrode unit furthermore being provided so as to be capable of being attached to and detached from each other by a plurality of connection parts; and the plurality of connection parts being provided with induction-electrode contact points for respectively connecting to the plurality of induction electrodes.

2. The mobile ECG apparatus according to claim 1, **characterized in that**: the connection parts are configured from connecting members provided to the ECG unit, and to-be-connected members provided to the electrode unit; and the induction-electrode contact points are provided to at least the connecting members.

3. The mobile ECG apparatus according either claim 1 or 2, **characterized in that** the ECG unit is provided with signal processing means for converting the body surface potential to an electrocardiographic signal, sampling means for sampling the electrocardiographic signal obtained by the signal processing means, and transmission means for wirelessly transmitting the sampled electrocardiographic signal to an analyzer.

4. The mobile ECG apparatus according to any one of claims 1-3, **characterized in that** the electrode unit is a handheld electrode unit that can be held in the hands.

5. The mobile ECG apparatus according to claim 4, **characterized in that** the handheld electrode unit is provided in a state such that at least two induction electrodes are set apart from each other.

6. The mobile ECG apparatus according to claim 5, **characterized in that** the at least two induction electrodes are separated by a distance of 100 mm or more.

7. The mobile ECG apparatus according to either claim 5 or 6, **characterized in that**: the handheld electrode unit is a substrate that can be held in the hands; and the at least two induction electrodes are provided on the two side-end surfaces of the substrate so as to sandwich the connection parts.

8. The mobile ECG apparatus according to claim 7, **characterized in that**: the substrate upper surface, which is substantially perpendicular to the right-side end surface on which is provided one induction electrode from among the induction electrodes, is provided with a cavity for induction from the thumb at a position where contact is made by the thumb of the right hand; and the substrate lower surface, which is on the opposite side from the substrate upper surface, is provided with a cavity for induction from the middle finger, the cavity being contacted by the middle finger of the right hand.

9. The mobile ECG apparatus according to claim 8, **characterized in that** the right-side end surface and the left-side end surface are both set in a shape so as to bend convexly outward.

10. The mobile ECG apparatus according to any one of claims 1-3, **characterized in that** the electrode unit is a body-surface-adhering electrode that can adhere to the body surface.
